(19) Europäisches Patentamt
European Patent Office
Office européen des brevets

(11) Veröffentlichungsnummer: **0 267 412 A1**

(12) **EUROPÄISCHE PATENTANMELDUNG**

(21) Anmeldenummer: **87114383.0**

(22) Anmeldetag: **02.10.87**

(51) Int. Cl.⁴: **C07D 471/14** , A01N 43/90 , C07F 9/65 , A01N 57/16 , //(C07D471/14,235:00,221:00,209:00)

(30) Priorität: **14.10.86 DE 3634952**

(43) Veröffentlichungstag der Anmeldung: **18.05.88 Patentblatt 88/20**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Draber, Wilfried, Dr.**
**In den Birken 81**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Strang, Robert Harry, Dr.**
**Unterdorfstrasse 6 A**
**D-4000 Düsseldorf 31(DE)**

(54) **Imidazo-pyrrolo-pyridin-Derivate.**

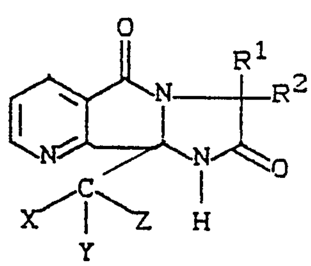

(I)

(57) in welcher
$R^1$ und $R^2$ unabhängig voneinander für Alkyl stehen oder gemeinsam für einen zweifach verknüpften Alkylenrest stehen,
X für Wasserstoff, Halogen oder Alkyl steht,
Y für Wasserstoff, Halogen, Cyano, Alkyl, für gegebenenfalls substituiertes Aryl, für Alkylsulfonyl, für Dialkoxyphosphoryl oder für einen Rest

$-\underset{\underset{O}{\|}}{C}-R^3$

steht und
Z für Cyano, Nitro oder für einen Rest

$-\underset{\underset{O}{\|}}{C}-R^3$ .

**EP 0 267 412 A1**

steht,
wobei
$R^3$ für Alkyl, Alkoxy, Cycloalkyl, Amino oder Alkoxycarbonyl steht,
ein Verfahren zu ihrer Herstellung und ihre Verwendung als Herbizide.

## Imidazo-pyrrolo-pyridin-Derivate

Die Erfindung betrifft neue Imidazo-pyrrolo-pyridin-Derivate, Verfahren zu ihrer Herstellung und ihre Verwendung als Herbizide.

Es ist bereits bekannt, daß bestimmte 2-(2-Imidazolin-2-yl)-pyridine, wie beispielsweise das 2-(4,4-Dimethyl-5-oxo-2-imidazolin-2-yl)-3-methoxycarbonyl-pyridin, herbizide Eigenschaften haben (vgl. z.B. EP-OS 41 623).

Die herbizide Wirkung dieser vorbekannten Verbindungen gegenüber Schadpflanzen ist jedoch nicht immer in allen Anwendungsbereichen völlig zufriedenstellend.

Es wurden neue Imidazo-pyrrolo-pyridin-Derivate der allgemeinen Formel (I),

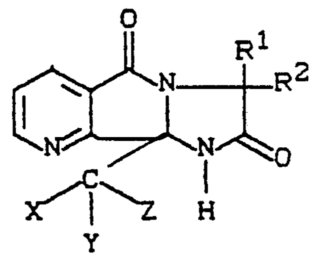

(I)

in welcher

$R^1$ und $R^2$ unabhängig voneinander für Alkyl stehen oder gemeinsam für einen zweifach verknüpften Alkylenrest stehen,

X für Wasserstoff, Halogen oder Alkyl steht,

Y für Wasserstoff, Halogen, Cyano, Alkyl, für gegebenenfalls substituiertes Aryl, für Alkylsulfonyl, für Dialkoxyphosphoryl oder für einen Rest

$-\underset{\underset{O}{\|}}{C}-R^3$

steht und

Z für Cyano, Nitro oder für einen Rest

$-\underset{\underset{O}{\|}}{C}-R^3$

steht,

wobei

$R^3$ für Alkyl, Alkoxy, Cycloalkyl, Amino oder Alkoxycarbonyl steht,

gefunden.

Weiterhin wurde gefunden, daß man die neuen Imidazo-pyrrolo-pyridin-Derivate der Formel (I),

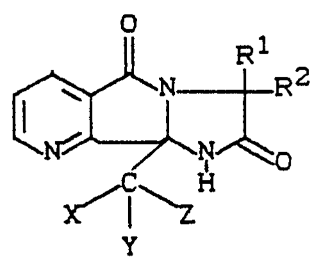

(I)

in welcher

$R^1$ und $R^2$ unabhängig voneinander für Alkyl stehen oder gemeinsam für einen zweifach verknüpften Alkylenrest stehen,

X für Wasserstoff, Halogen oder Alkyl steht,

Y für Wasserstoff, Halogen, Cyano, Alkyl, für gegebenenfalls substituiertes Aryl, für Alkylsulfonyl, für Dialkoxyphosporyl oder für einen Rest

$-\underset{\underset{O}{\|}}{C}-R^3$

steht und

Z für Cyano, Nitro oder für einen Rest
$-\underset{\underset{O}{\|}}{C}-R^3$
steht,
wobei
$R^3$ für Alkyl, Alkoxy, Cycloalkyl, Amino oder Alkoxycarbonyl steht,
erhält, wenn man Imidazo-pyrrolo-pyridine der Formel (II),

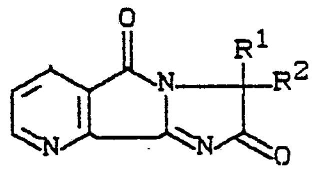

(II)

in welcher
$R^1$ und $R^2$ die oben angegebene Bedeutung haben,
mit substituierten Methan-Derivaten der Formel (III),

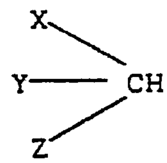

(III)

in welcher
X, Y und Z die oben angegebene Bedeutung haben,
in Gegenwart eines basischen Reaktionshilfsmittels und gegebenenfalls in Gegenwart eines Verdünnungsmittels umsetzt.

Schließlich wurde gefunden, daß die neuen Imidazo-pyrrolo-pyridin-Derivate der Formel (I) herbizide Eigenschaften besitzen.

Überraschenderweise zeigen die erfindungsgemäßen Imidazo-pyrrolo-pyridin-Derivate der Formel (I) eine erheblich höhere herbizide Potenz als die aus dem Stand der Technik bekannten 2-(2-Imidazolin-2-yl)-pyridine, wie beispielsweise das 2-(4,4-Dimethyl-5-oxo-2-imidazolin-2-yl)-3-methoxycarbonyl-pyridin, welche chemisch und wirkungsmäßig naheliegende Verbindungen sind.

Die erfindungsgemäßen Imidazo-pyrrolo-pyridin-Derivate sind durch die Formel (I) allgemein definiert. Bevorzugt sind Verbindungen der Formel (I), bei welchen
$R^1$ und $R^2$ unabhängig voneinander für geradkettiges oder verzweigtes Alkyl mit 1 bis 8 Kohlenstoffatomen stehen oder gemeinsam für einen zweifach verknüpften geradkettigen oder verzweigten Alkylenrest mit 3 bis 10 Kohlenstoffatomen stehen,
X für Wasserstoff, Fluor, Chlor, Brom oder Iod oder für geradkettiges oder verzweigtes Alkyl mit 1 bis 8 Kohlenstoffatomen steht,
Y für Wasserstoff, Fluor, Chlor, Brom, Iod, Cyano, für geradkettiges oder verzweigtes Alkyl mit 1 bis 8 Kohlenstoffatomen oder für ge gebenenfalls einfach oder mehrfach, gleich oder verschieden substituiertes Phenyl steht, wobei als Substituenten infrage kommen: Halogen, Cyano, Nitro sowie jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen; außerdem für Alkylsulfonyl mit 1 bis 4 Kohlenstoffatomen im Alkylteil oder für Dialkoxyphosphoryl mit jeweils 1 bis 4 Kohlenstoffatomen in den einzelnen Alkoxyteilen oder für einen Rest
$-\underset{\underset{O}{\|}}{C}-R^3$
steht und
Z für Cyano, Nitro oder für einen Rest
$-\underset{\underset{O}{\|}}{C}-R^3$
steht,
wobei

$R^3$ für jeweils geradkettiges oder verzweigtes Alkyl, Alkoxy oder Alkoxycarbonyl mit jeweils 1 bis 6 Kohlenstoffatomen,für Cycloalkyl mit 3 bis 7 Kohlenstoffatomen oder für Amino steht.

Besonders bevorzugt sind Verbindungen der Formel (I), bei welchen

$R^1$ und $R^2$ unabhängig voneinander für Methyl, Ethyl, n-oder i-Propyl sowie n-, i-, s-oder t-Butyl stehen oder gemeinsam für einen jeweils zweifach verknüpften 1,3-Propandiylrest, einen 1,4-Butandiylrest oder 1,5-Pentandiylrest stehen,

X für Wasserstoff, Fluor, Chlor, Brom, Methyl, Ethyl, n-oder i-Propyl, n-, i-, s-oder t-Butyl steht,

Y für Wasserstoff, Fluor, Chlor, Brom, Cyano, Methyl, Ethyl, n-oder i-Propyl, n-, i-, s-oder t-Butyl oder für gegebenenfalls ein-bis dreifach gleich oder verschieden substituiertes Phenyl steht, wobei als Substituenten infrage kommen: Fluor, Chlor, Brom, Cyano, Nitro, Methyl, Ethyl oder Trifluormethyl; außerdem für Methylsulfonyl, Ethylsulfonyl, Dimethoxyphosphoryl, Diethoxyphosphoryl oder für einen Rest

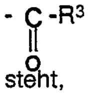

steht,

Z für Cyano, Nitro oder für einen Rest

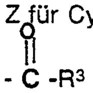
steht,

wobei

$R^3$ für Methyl, Ethyl, n-oder i-Propyl, n-, i-, s-oder t-Butyl, Methoxy, Ethoxy, n-oder i-Propoxy, Methoxycarbonyl, Ethoxycarbonyl, Cyclopropyl, Cyclohexyl oder Amino steht.

Im einzelnen seien die bei den Herstellungsbeispielen aufgeführten Verbindungen der allgemeinen Formel (I) genannt.

Verwendet man beispielsweise 3-Isopropyl-3-methyl-5H-imidazo-[1′, 2′:1,2]-pyrrolo-[3,4-b]-pyridin-2-(3H),5-dion und Cyanessigsäuremethylester als Ausgangsstoffe, so läßt sich der Reaktionsablauf des erfindungsgemäßen Verfahrens durch das folgende Formelschema darstellen:

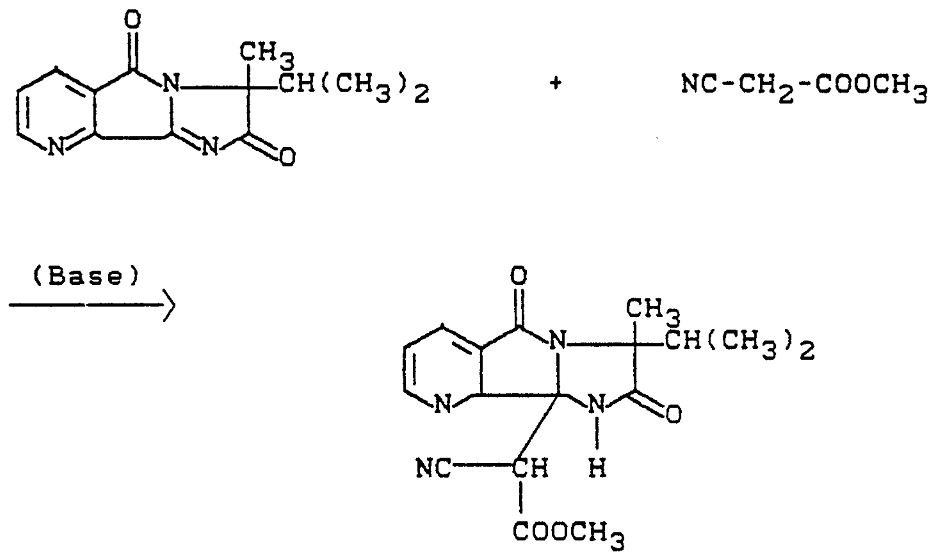


Die zur Durchführung des erfindungsgemäßen Verfahrens als Ausgangsstoffe benötigten Imidazopyrrolopyridine sind durch die Formel (II) allgemein definiert. In dieser Formel (II) stehen $R^1$ und $R^2$ vorzugsweise für diejenigen Reste, die schon im Zusammenhang mit der Beschreibung der erfindungsgemäßen Stoffe der Formel (I) als bevorzugt für diese Substituenten genannt wurden.

Die Imidazo-pyrrolo-pyridine der Formel (II) und Verfahren zu ihrer Herstellung sind bekannt (vgl. z.B. EP-OS 41 623).

Die zur Durchführung des erfindungsgemäßen Verfahrens weiterhin als Ausgangsstoffe benötigten substituierten Methan-Derivate sind durch die Formel (III) allgemein definiert. In dieser Formel (III) stehen X, Y und Z vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Methan-Derivate der Formel (III) sind allgemein bekannte Verbindungen der organischen Chemie.

Als Verdünnungsmittel zur Durchführung des erfindungsgemäßen Verfahrens kommen inerte organische

Lösungsmittel infrage. Hierzu gehören insbesondere aliphatische, alicyclische oder aromatische Kohlenwaserstoffe, wie Benzin, Benzol, Toluol, Xylol, Petrolether, Hexan, Cyclohexan, Ether, wie Diethylether, Dioxan, Tetrahydrofuran oder Ethylenglykoldimethyl-oder diethylether oder Alkohole wie Isopropanol oder t-Butanol.

Das erfindungsgemäße Verfahren wird in Gegenwart eines geeigneten basischen Reaktionshilfsmittels durchgeführt. Als solche kommen alle üblichen anorganischen oder organischen Basen infrage. Mit besonderem Vorzug verwendet man Alkalimetallalkoholate, wie beispielsweise Natriummethylat oder -ethylat oder Kalium-t-butylat.

Die Reaktionstemperaturen können bei Durchführung des erfindungsgemäßen Verfahrens in einem größeren Bereich variiert werden. Im allgemeinen arbeitet man bei Temperaturen zwischen -20°C und +60°C, vorzugsweise bei Temperaturen zwischen 0°C und 30°C.

Zur Durchführung des erfindungsgemäßen Verfahren setzt man pro Mol an Imidazo-pyrrolo-pyridin-Derivat der Formel (II) im allgemeinen 1,0 bis 1,5 Mol, vorzugsweise äquimolare Mengen an substituierten Methan-Derivat der Formel (III) und 0,01 bis 0,5 Mol, vorzugsweise 0,1 bis 0,2 Mol an basischem Reaktionshilfsmittel ein. Zur Aufarbeitung fällt man das Produkt mit einem geeigneten organischen Lösungsmittel aus, trocknet und reinigt falls erforderlich durch Umfällen oder Umkristallisieren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Vor-und Nachauflaufunkrautbekämpfung von mono-und dikotylen Unkräutern einsetzen. In entsprechenden Aufwandmengen besitzen die erfindungsgemäßen Wirkstoffe auch eine wachstumsregulatorische Wirkung und lassen sich beispielsweise als Baumwolldefoliantien verwenden.

Darüberhinaus besitzen die erfindungsgemäßen Stoffe auch eine gute fungizide und bakterizide Wirkung und lassen sich in entsprechend verringerten Aufwandmengen auch zur Bekämpfung von Reiskrankheiten wie beispielsweise gegen den Erreger der Reisfleckenkrankheit (Pyricularia oryzae) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und syn thetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treib gase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farb stoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werde.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-di-methyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-di-methylethyl)-3-methylthio-1,2,4-triazin-5(4H)-on zur Unkrautbekämpfung in Sojabohnen, in Frage.

Auch Mischungen mit 2-Chlor-4,6-bis-(ethylamino)-1,3,5-triazin, 5-Amino-1,2,4-triazol, N-Phosphonomethylglycin, N,N-Dimethyl-N'-(3,4-dichlorphenyl)-harnstoff, N,N'-Dimethyl-N-(5-ethylsulfonyl-1,3,4-thiadiazol-2-yl)-harnstoff, 2,4-Dichlorphenoxyessigsäure, 2,4-Dichlorphenoxypropionsäure, (2-Methyl-4-chlorphenoxy)-essig säure, (4-Chlor-2-methylphenoxy)-propionsäure, 1,1'-Dimethyl-4,4'-bipyridyliumdichlorid, Ammonium-(3-amino-3-carboxypropyl)-methyl-phoshinat oder Methyl-2-{[(4,6-dimethyl-2-pyrimidinyl)-aminocarbonyl]-aminosulfonyl}-benzoat sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmittel ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,05 und 10 kg Wirkstoff pro Hektar Bodenfläche, vorzugsweise zwischen 0,1 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

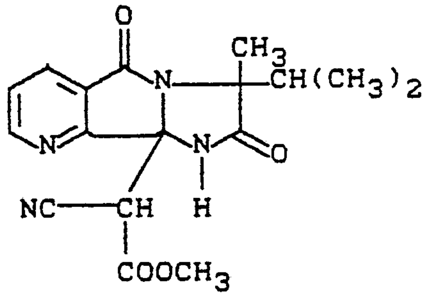

Zu 243 g (1 Mol) 3-Isopropyl-3-methyl-5H-imidazo[1', 2':1,2] pyrrolo[3,4-b]-pyridin-2-(3H),5-dion in 500 ml Toluol und 2500 ml t-Butanol gibt man zunächst 100 g (1 mol) Cyanessigsäuremethylester und anschließend 20 g (0,18 mol) Kalium-t-butylat, rührt 6 Stunden bei Raumtemperatur, gibt 500 ml Toluol zu und rührt weitere 15 Stunden bei Raumtemperatur. Der entstandene Niederschlag wird abgesaugt, mit Isopropanol/Ether gewaschen und getrocknet.

Man erhält 273,1 g (80 % der Theorie) an Verbindung der oben angegebenen Formel vom Schmelzpunkt 128°C - 131°C (Zers.).

In entsprechender Weise und gemäß den allgemeinen Angaben zur Herstellung erhält man die folgenden Imidazo-pyrrolo-pyridin-Derivate der allgemeinen Formel (I):

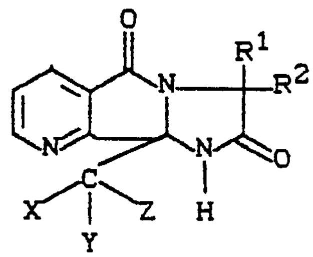

(I)

Tabelle 1

| Bsp. Nr. | $R^1$ | $R^2$ | 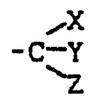 | Schmelzpunkt /° C |
|---|---|---|---|---|
| 2 | $CH_3$ | $(CH_3)_2CH-$ | 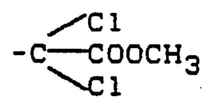 | 184(Zers.) |
| 3 | $CH_3$ | $(CH_3)_2CH-$ | 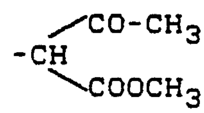 | 171(Zers.) |
| 4 | $CH_3$ | $(CH_3)_2CH-$ | 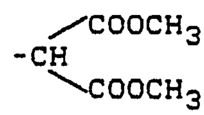 | 107 |
| 5 | $CH_3$ | $C_2H_5$ | 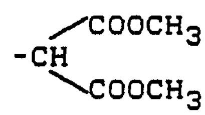 | 128-130 |
| 6 | $-CH_2-CH_2-CH_2-CH_2-CH_2-$ | | 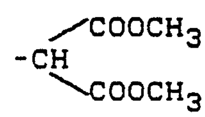 | 111 |
| 7 | $CH_3$ | $C_2H_5$ | 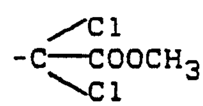 | 184 |
| 8 | $CH_3$ | $(CH_3)_2CH-$ | 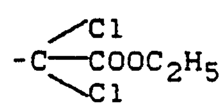 | 173 |

Tabelle 1 (Fortsetzung)

| Bsp. Nr. | $R^1$ | $R^2$ | 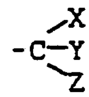 | Schmelzpunkt /° C |
|---|---|---|---|---|
| 9 | $CH_3$ | $(CH_3)_2CH-$ | 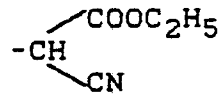 | 126 |
| 10 | $CH_3$ | $(CH_3)_2CH-$ | 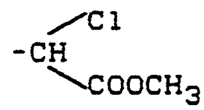 | 143 |
| 11 | $CH_3$ | $(CH_3)_2CH-$ | $-CH_2-NO_2$ | 132-134 |
| 12 | $CH_3$ | $(CH_3)_2CH-$ | $-CH_2-CO-CH_3$ | 158 |
| 13 | $CH_3$ | $(CH_3)_2CH-$ | 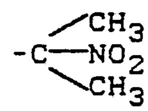 | 143 |
| 14 | $CH_3$ | $(CH_3)_2CH-$ | 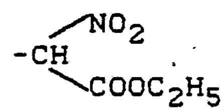 | 117 |
| 15 | $CH_3$ | $(CH_3)_2CH-$ | $-CH_2-CO-CH(CH_3)_2$ | 146 |
| 16 | $CH_3$ | $(CH_3)_2CH-$ | 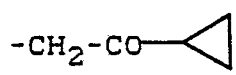 | 179 |
| 17 | $CH_3$ | $(CH_3)_2CH-$ | $-CH_2-CO-C_2H_5$ | 164 |

Anwendungsbeipiele:

In den folgenden Anwendungsbeispielen wurde die nachstehend aufgeführte Verbindung als Vergleichssubstanz herangezogen:

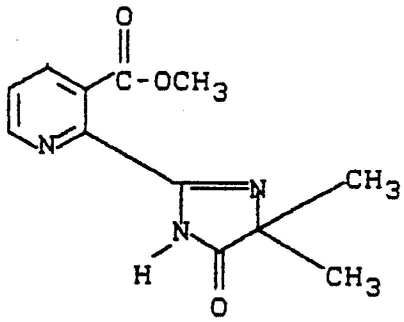

(A)

2-(4,4-Dimethyl-5-oxo-2-imidazolin-2-yl)-3-methoxycarbonyl-pyridin.
(bekannt aus EP-OS 41 623)

Beispiel A

Post-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber der Vergleichssubstanz (A) zeigen in diesem Test beispielsweise die Verbindungen gemäß den Herstellungsbeispielen 1, 2, 3 und 4.

**Ansprüche**

1. Imidazo-pyrrolo-pyridin-Derivate der Formel (I),

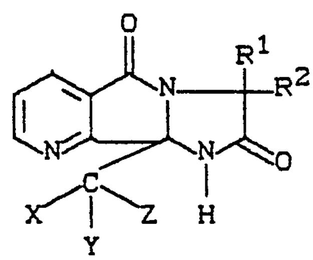

(I)

in welcher
$R^1$ und $R^2$ unabhängig voneinander für Alkyl stehen oder gemeinsam für einen zweifach verknüpften Alkylenrest stehen,
X für Wasserstoff, Halogen oder Alkyl steht,
Y für Wasserstoff, Halogen, Cyano, Alkyl, für gegebenenfalls substituiertes Aryl, für Alkylsulfonyl, für Dialkoxyphosphoryl oder für einen Rest

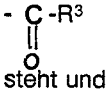

steht und

Z für Cyano, Nitro oder für einen Rest

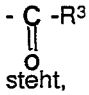

steht,

wobei

$R^3$ für Alkyl, Alkoxy, Cycloalkyl, Amino oder Alkoxycarbonyl steht.

2. Imidazo-pyrrolo-pyridin-Derivate der Formel (I) gemäß Anspruch 1, in welcher

$R^1$ und $R^2$ unabhängig voneinander für geradkettiges oder verzweigtes Alkyl mit 1 bis 8 Kohlenstoffatomen stehen oder gemeinsam für einen zweifach verknüpften geradkettigen oder verzweigten Alkylenrest mit 3 bis 10 Kohlenstoffatomen stehen,

X für Wasserstoff, Fluor, Chlor, Brom oder Iod oder für geradkettiges oder verzweigtes Alkyl mit 1 bis 8 Kohlenstoffatomen steht,

Y für Wasserstoff, Fluor, Chlor, Brom, Iod, Cyano, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten infrage kommen: Halogen, Cyano, Nitro sowie jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen; außerdem für Alkylsulfonyl mit 1 bis 4 Kohlenstoffatomen im Alkylteil oder für Dialkoxyphosphoryl mit jeweils 1 bis 4 Kohlenstoffatomen in den einzelnen Alkoxyteilen oder für einen Rest

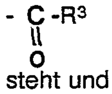

steht und

Z für Cyano, Nitro oder für einen Rest

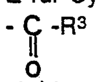

steht,

wobei

$R^3$ für jeweils geradkettiges oder verzweigtes Alkyl, Alkoxy oder Alkoxycarbonyl mit jeweils 1 bis 6 Kohlenstoffatomen, für Cycloalkyl mit 3 bis 7 Kohlenstoffatomen oder für Amino steht.

3. Imidazo-pyrrolo-pyridin-Derivate der Formel (I) gemäß Anspruch 1, in welcher

$R^1$ und $R^2$ unabhängig voneinander für Methyl, Ethyl, n-oder i-Propyl sowie n-, i-, s-oder t-Butyl stehen oder gemeinsam für einen jeweils zweifach verknüpften 1,3-Propan diylrest, einen 1,4-Butandiylrest oder 1,5-Pentandiylrest stehen

X für Wasserstoff, Fluor, Chlor, Brom, Methyl, Ethyl, n-oder i-Propyl, n-, i-, s-oder t-Butyl steht,

Y für Wasserstoff, Fluor, Chlor, Brom, Cyano, Methyl, Ethyl, n-oder i-Propyl, n-, i-, s-oder t-Butyl oder für gegebenenfalls ein-bis dreifach gleich oder verschieden substituiertes Phenyl steht, wobei als Substituenten infrage kommen: Fluor, Chlor, Brom, Cyano, Nitro, Methyl, Ethyl oder Trifluormethyl; außerdem für Methylsulfonyl, Ethylsulfonyl, Dimethoxyphosphoryl, Diethoxyphosphoryl oder für einen Rest

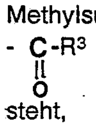

steht,

Z für Cyano, Nitro oder für einen Rest

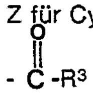 steht,

wobei

$R^3$ für Methyl, Ethyl, n-oder i-Propyl, n-, i-, s-oder t-Butyl, Methoxy, Ethoxy, n-oder i-Propoxy, Methoxycarbonyl, Ethoxycarbonyl, Cyclopropyl, Cyclohexyl oder Amino steht.

4. Verfahren zur Herstellung von Imidazo-pyrrolo-pyridin-Derivaten der Formel (I),

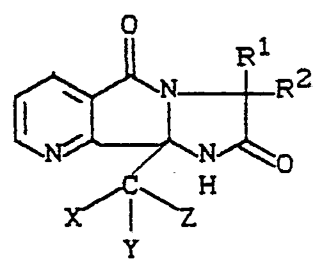

(I)

in welcher
$R^1$ und $R^2$ unabhängig voneinander für Alkyl stehen oder gemeinsam für einen zweifach verknüpften Alkylenrest stehen,
X für Wasserstoff, Halogen oder Alkyl steht,
Y für Wasserstoff, Halogen, Cyano, Alkyl, für gegebenenfalls substituiertes Aryl, für Alkylsulfonyl, für Dialkoxyphosporyl oder für einen Rest

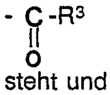

steht und
Z für Cyano, Nitro oder für einen Rest

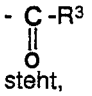

steht,
wobei
$R^3$ für Alkyl, Alkoxy, Cycloalkyl, Amino oder Alkoxycarbonyl steht,
dadurch gekennzeichnet, daß man Imidazo-pyrrolo-pyridine der Formel (II),

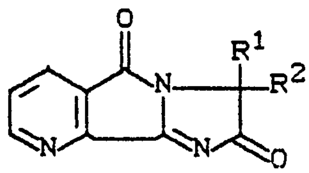

(II)

in welcher
$R^1$ und $R^2$ die oben angegebene Bedeutung haben,
mit substituierten Methan-Derivaten der Formel (III),

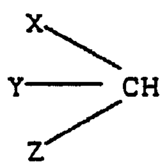

(III)

in welcher
X, Y und Z die oben angegebene Bedeutung haben,
in Gegenwart eines basischen Reaktionshilfsmittels und gegebenenfalls in Gegenwart eines Verdünnungsmittels umsetzt.

5. Herbizide Mittel, gekennzeichnet durch einen Gehalt an mindestens einem Imidazo-pyrrolo-pyridin-Derivat der Formel (I) gemäß den Ansprüchen 1 bis 4.

6. Verfahren zur Bekämpfung von Unkräutern, dadurch gekennzeichnet, daß man Imidazo-pyrrolo-pyridin-Derivate der Formel (I) gemäß den Ansprüchen 1 bis 4 auf die Unkräuter und/oder ihren Lebensraum einwirken läßt.

7. Verwendung von Imidazo-pyrrolo-pyridin-Derivaten der Formel (I) gemäß den Ansprüchen 1 bis 4 zur Bekämpfung von Unkräutern.

8. Verfahren zur Herstellung von herbiziden Mitteln, dadurch gekennzeichnet, daß man Imidazo-pyrrolo-pyridin-Derivate der Formel (I) gemäß den Ansprüchen 1 bis 4 mit Streckmitteln und/oder oberflächenaktiven Substanzen vermischt.

9. Die Verbindung gemäß Anspruch 1

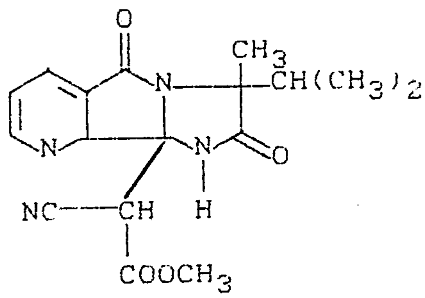

10. Die Verbindung gemäß Anspruch 1

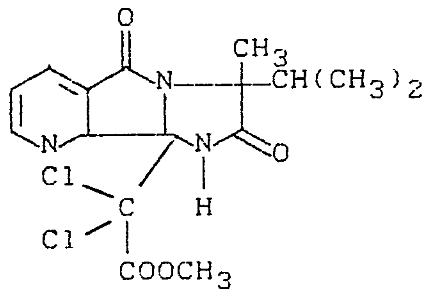

Europäisches Patentamt

# EUROPÄISCHER RECHERCHENBERICHT

Nummer der Anmeldung

EP 87 11 4383

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | EP-A-0 195 745 (CIBA-GEIGY)<br>* Ansprüche 1,20 *<br>----- | 1,5 | C 07 D 471/14<br>A 01 N 43/90<br>C 07 F 9/65<br>A 01 N 57/16 //<br>(C 07 D 471/14<br>C 07 D 235:00<br>C 07 D 221:00<br>C 07 D 209:00 ) |
| | | | RECHERCHIERTE SACHGEBIETE (Int. Cl.4) |
| | | | C 07 D 471/00 |

Der vorliegende Recherchenbericht wurde für alle Patentansprüche erstellt

| Recherchenort | Abschlußdatum der Recherche | Prüfer |
|---|---|---|
| DEN HAAG | 20-01-1988 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)